# EUROPEAN PATENT SPECIFICATION

(11) **EP 2 038 576 B1**
(45) Date of publication and mention of the grant of the patent: **21.08.2013**
(21) Application number: 07747140.7
(22) Date of filing: 27.06.2007
(51) Int. Cl.: F21S 2/00, A01G 7/04, H05B 33/08

(54) **ARTIFICIAL SOLAR LIGHT SYSTEM USING A LIGHT EMITTING DIODE**
KÜNSTLICHES SONNENLICHTSYSTEM MIT LEUCHTDIODE
SYSTEME ARTIFICIEL DE LUMIERE SOLAIRE UTILISANT UNE DIODE ELECTROLUMINESCENTE

(30) Priority: 28.06.2006 KR 20060058827
(43) Date of publication of application: 25.03.2009
(73) Proprietor: Seoul Semiconductor Co., Ltd., Seoul 153-801 (KR)
(72) Inventor: SON, Won Kuk, Gyeonggi-do 425-868 (KR); KANG, Seok Jin, Gyeonggi-do 425-851 (KR); CHO, Won, Gyeonggi-do 425-876 (KR)
(74) Representative: Scheele, Friedrich
(86) International application number: PCT/KR2007/003118
(87) International publication number: WO 2008/002073

(56) References cited:
- US-A1- 2005 030 744
- US-A1- 2005 052 378
- US-A1- 2006 022 214
- US-B2- 6 890 085

## Description

### Technical Field

The present invention relates to an artificial solar light system, and more particularly, to an artificial solar light system using light emitting diodes.

### Background Art

Generally, in places into which solar light is difficult to come, i.e., basements or other closed spaces, various symptoms of side effects (such as undergrowth, skin diseases, and psychological anxieties) may occur due to an insufficient amount of sunshine. Conventionally, as attempt to solve this problem, an artificial solar light system for generating artificial solar light, and a solar light tracking system for allowing real solar light from an outdoor space to directly come into an indoor space through an optical fiber or the like have been constructed.

In the artificial solar light system of the conventional techniques described above, artificial light sources such as incandescent lamps, metal halide lamps or xenon lamps are constructed into a certain module to manufacture a single artificial solar light irradiation apparatus. Since the incandescent lamp has a better color rendering property than any other current light source, it can show spectrum effects of solar light that reaches the earth's surface on a clear sunny day. However, since the incandescent lamp has a color temperature of about 3200K, it is difficult to represent characteristics of a color temperature of real solar light (from about 3000K to 7000K) according to time zones. Further, since color temperature indexes of the metal halide lamp and the xenon lamp are also limited to about 5000K and 6000K, respectively, it is impossible to represent the same light emission effects as the sun with time.

Moreover, in the solar light tracking system, a travel path of the sun is tracked, and solar light is collected and received by optical lenses in real time and then introduced into an indoor space through the glass optical fiber. In the solar light tracking system, the plurality of optical lenses are arranged on a solar light collecting surface , a photo sensor is positioned at the center of a lens module, and a program is installed in a microprocessor to automatically rotate a solar light collecting surface module in real time according to the travel path of the sun. The conventional solar light tracking system described above has advantages in that the solar light tracking system can receive the energy of real solar light as it is and a harmful wavelength can be removed from the solar light by using a specific filter. However, there is a disadvantage in that the solar light tracking system cannot be used in the rainy season or on cloudy days. Further, since the optical lenses should be positioned outdoors to receive the solar light, they require special care in maintenance. Further, the solar light should be collected by the optical lenses and then transmitted into an indoor space through the optical fiber. In this case, a glass optical fiber with a superior thermal property rather than an inexpensive resin-based optical fiber should be used as the optical fiber. However, since a small-sized glass optical fiber on the order of microns causes great loss, there is a problem In that an expensive glass optical fiber with a diameter of 1 mm or more should be employed. Moreover, since the optical lenses are typically mounted on the roof of a building, costs accordingly increase due to the increased length of the glass optical fiber to be used and a loss fraction also increases due to an increased distance, resulting in limitations on the installation and use thereof.

US 2006/022214 A1 discloses all features of the preamble of present claim 1.

### Disclosure of invention

### Technical Problem

The present invention is conceived to solve the aforementioned problems in the prior art. Accordingly, an object of the present invention is to provide an artificial solar light system using light emitting diodes, which can represent the same light emission effects as the sun with time.

Further, another object of the present invention is to provide an artificial solar light system using light emitting diodes, which is inexpensive and is not affected by positions or climate.

### Technical Solution

To achieve these objects, the present invention provides an artificial solar light system using light emitting diodes, comprising a plurality of light emitting diode modules having different color temperatures: and a control unit for controlling the plurality of light emitting diode modules, a memory unit configured to store data on changes in a color temperature and optical properties of the sun with time; and a microprocessor configured to control currents applied to the respective light emitting diode modules having different color temperatures by using the data on changes in the color temperature and optical properties of the sun with time, which have been stored in the memory unit, wherein the memory unit further includes solar coordinate system information according to periods of earth's rotation and revolution, and the microprocessor is configured to control the light emitting diode modules by using the data on changes in the color temperature and optical properties of the sun with time and the solar coordinate system information, which have been stored in the memory unit, the control unit further comprises a global positioning system for identifying a position of the artificial solar light system; and the microprocessor is configured to control the light emitting diode modules by using the data on changes in the color temperature and optical properties of the sun with time and the solar coordinate system information, which have been stored in the memory unit, depending on the position identified by the global positioning system.

The artificial solar light system of the present invention may further comprise a diffusion globe arranged in front of the plurality of light emitting diode modules so as to mix light emitted from the plurality of light emitting diode modules and to irradiate the mixed light.

At this time, the plurality of light emitting diode modules may be alternately arranged.

The plurality of light emitting diode modules may have a color temperature of 3000K to 7000K. The control unit may comprise a memory unit for storing data on changes in a color temperature and optical properties of the sun with time; and a microprocessor for controlling currents applied to the respective light emitting diode modules having different color temperatures by using the data on changes in the color temperature and optical properties of the sun with time, which have been stored in the memory unit.

At this time, the microprocessor may receive information on a position and controls the light emitting diode modules by using the data on changes in the color temperature and optical properties of the sun with time and the solar coordinate system information, which have been stored in the memory unit, depending on the position information.

### Advantageous Effects

According to the present invention described above, it is possible to provide an artificial solar light system using light emitting diodes, which can represent the same light emission effects as the sun with time. [15] Further, the present invention can provide an artificial solar light system using light emitting diodes, which is inexpensive and is not affected by positions or climate.

### Brief Description of the Drawings

Fig.1 is a conceptual view showing an artificial solar light system using light emitting diodes according to a first embodiment of the present invention.
Fig. 2 is a plan view showing a light source module in the first embodiment of the present invention.
Fig. 3 is a conceptual view showing an artificial solar light system using light emitting diodes according to a second embodiment of the present invention.

### <Explanation of Reference Numerals for Main Portions in Drawings>

100: First light emitting diode module
200: Second light emitting diode module
300: Base plate 400: Light source module
500: Diffusion globe 600: Control unit
620: Microprocessor 640: memory unit
660: Global positioning system

### Best Mode for Carrying Out the Invention

The present invention is not limited to embodiments set forth below but can be implemented in different forms. The embodiments are provided for complete disclosure of the present invention and for fully conveying the scope of the present invention to those skilled in the art. Like reference numerals in the drawings indicate like elements.

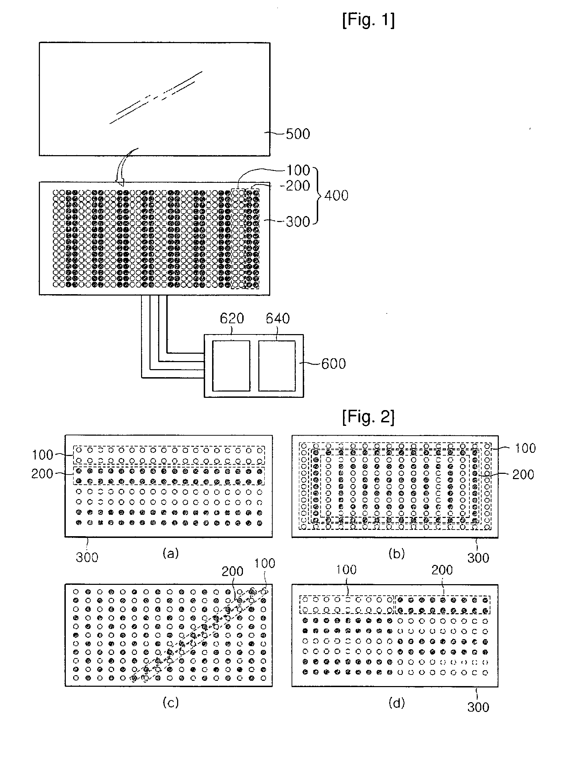

Fig. 1 is a conceptual view showing an artificial solar light system using light emitting diodes according to a first embodiment of the present invention.

As shown in Fig. 1, the artificial solar light system using light emitting diodes according to the first embodiment of the present invention includes a light source module 400, a diffusion globe 500 provided on the light source module 400, and a control unit 600 for controlling the light source module 400.

The light source module 400 serves as a light source for the artificial solar light system using light emitting diodes according to the present invention, and includes a base plate 300 and first and second light emitting diode modules 100 and 200 that are mounted on the base plate 300.

The base plate 300 is used for fixedly mounting the first and second light emitting diode modules 100 and 200 thereon, and may be constructed to be in the form of a conventional substrate, i.e., to have a structure with an electrode pattern formed on an insulator. The first and second light emitting diode modules 100 and 200 are mounted on the base plate 300 so as to be connected to the electrode pattern that in turn is connected to an external power supply, so that the first and second light emitting diode modules 100 and 200 can be operated. The shape of the base plate 300 may be variously changed depending on the object and use of the artificial solar light system using light emitting diodes. That is, although the base plate 300 is made in the form of a rectangular plate in this embodiment, the present invention is not limited thereto. The base plate 300 may be in the form of a circular or polygonal plate. Alternatively, the base plate 300 may be in the form of a cylindrical or polygonal post, or a sphere. The shape of the base plate 300 is not limited thereto so far as it meets the object of the present invention.

The first and second light emitting diode modules 100 and 200 include first and second light emitting diode modules 100 and 200 having a color temperature of 3000K to 7000K.

At this time, the first light emitting diode module 100 may include a plurality of light emitting diodes having a color temperature of about 3000K to 5000K, while the second light emitting diode module 200 may include a plurality of light emitting diodes having a color temperature of about 5000K to 7000K. However, the present invention is not limited thereto but may further include a third light emitting diode module. That is, for example, it is possible to employ a first light emitting diode module having a color temperature of about 3000K to 4500K, a second light emitting diode module having a color temperature of about 4500K to 6000K, and a third light emitting diode module having a color temperature of about 6000K to 7000K. As described above, the present invention may employ at least two light emitting diode modules of which color temperatures range from 3000K to 7000 and are different from each other. At this time, the respective color temperature ranges of the light emitting diode modules may overlap with each other at their boundary regions.

Each of light emitting diodes of the first and second light emitting diode modules 100 and 200 described above includes a light emitting chip and a phosphor. The light emitting chip and the phosphor may be implemented in various manners. For example, the light emitting diode may include a single blue light emitting chip capable of blue light and a yellow phosphor capable of emitting yellow light. That is, the blue light emitted by the blue light emitting chip and the yellow light obtained through wavelength conversion of the phosphor are mixed to implement white light.

Further, the light emitting diode may include a single blue light emitting chip capable of emitting blue light, and a green phosphor capable of emitting green light, and an orange phosphor capable of emitting orange light. At this time, the blue light emitted by the blue light emitting chip, and the green and orange light obtained through wavelength conversion of the phosphors are mixed to implement white light. In this case, there is an advantage in that better color rendering property can be obtained than the light emitting diode including the blue light emitting chip and the yellow phosphor. That is, it is possible to improve the color rendering property by using a light emitting chip and a plurality of kinds of phosphors with various light emission peaks. If a plurality of kinds of phosphors are used, it is possible to implement white light having a different color temperature and color rendering property depending on not only the composition of each of the phosphors but also the composition ratio of the phosphors. It is preferred that a blue light emitting chip or an ultraviolet light emitting chip be used as the light emitting chip in the present invention.

A series of materials with various light emission peak ranges, e.g., silicate-based phosphors with a light emission peak range from green to red, may be used as the phosphor in the present invention. That is, light emitted by the light emitting chip can be used as an excitation source for implementing various colors, thereby implementing white light with a different optical spectrum and color temperature characteristic. Further, if a plurality of kinds of phosphors are included, an identical series of materials may be used to minimize influences among the phosphors.

The first light emitting diode modules 100 and the second light emitting diode modules 200 for certain light that is distinguished from each other according to color temperatures as described above are alternately arranged to construct the single light source module 400. When the light source module 400 is constructed, the light emitting diode modules with certain color temperature ranges are classified according to predetermined range intervals as described above, and modularized respectively. In this embodiment, the first and second light emitting diode modules 100 and 200 are arranged in two columns as shown in Fig. 1. However, the first and second light emitting diode modules are not limited thereto but may be arranged in more or less than two columns. Further, the first and second light emitting diode modules 100 and 200 are alternately arranged in Fig. 1 but are not limited thereto. That is, as shown in Fig. 2 (a), the first and second light emitting diode modules 100 and 200 may be arranged in rows rather than in columns. Alternatively, as shown in Fig. 2 (b), the first and second light emitting diode modules 100 and 200 may be arranged in the form of bands. Alternatively, the first and second light emitting diode modules 100 and 200 may be arranged in a diagonal direction as shown in Fig. 2 (c), or may be arranged alternately in both row and column directions as shown in Fig. 2 (d). Furthermore, although not shown in the figures, a plurality of first light emitting diode modules 100 may be arranged at one side of the base plate 300 while a plurality of second light emitting diode modules 200 may be arranged at the other side of the base plate 300, or the first and second light emitting diode modules 100 and 200 may be randomly arranged. However, it is preferred that identical numbers of the first and second light emitting diode modules 100 and 200 be alternately arranged so that the artificial solar light system can represent a constant color temperature as a whole.

The control unit 600 is to accurately operate the light source module 400, and includes a memory unit 640 for storing data on changes in the color temperature and optical properties of the sun with time, and a microprocessor 620 for controlling the respective light emitting diode modules with different color temperatures by using the data. That is, the microprocessor 620 performs control of currents for the respective light emitting diode modules so as to respond to the changes in the color temperature and optical properties of the sun with time. Such current control allows the light source module 400 to emit light that has properties similar to those of spectrum variations of real solar light. However, the present invention is not limited thereto. A switchover to a manual mode may be performed if a user wants to use a wavelength in a specific color temperature range.

Further, the artificial solar light system of the present invention can be changed in size depending on a use of the light source module 400. That is, the size of the artificial solar light system can be increased or decreased according to both the number of the light emitting diode modules and the number of the light emitting diodes provided in the light source module 400.

The diffusion globe 500 is to mix light emitted from the light emitting diode modules, and is arranged in front of the light emitting diode modules. The diffusion globe 500 is made of a material having a superior light transmission property, and functions to mix differences in color senses of the respective light emitting diode modules having different color temperatures so as to smooth differences in chromaticity. The diffusion globe 500 described above may be eliminated depending on the object and use of the artificial solar light system.

As described above, a plurality of light emitting diodes with a color temperature of about 3000K to 5000K are used for the first light emitting diode module 100, and a plurality of light emitting diodes with a color temperature of about 5000K to 7000K are used for the second light emitting diode module 200, so that the artificial solar light system of the present invention has a color temperature distribution of about 3000K to 7000K, which is similar to that of the sun. In the light emitting diode modules with the color temperature distribution described above, light emitting diode modules with similar color temperatures are grouped and provided with an independent power line.

In the light source modules provided with the independent power line as described above, the magnitude of a current is adjusted such that a higher current is applied to light emitting diodes having a color temperature property corresponding to a change in sunshine while a lower current is applied to the other light emitting diodes, thereby controlling the overall color temperature. That is, for example, in the morning, a higher current is applied to the first light emitting diode module 100 with a lower color temperature while a lower current is applied to the second light emitting diode module 200. Meanwhile, at noon, an identical current is applied to the first and second light emitting diode modules 100 and 200. Further, in the afternoon, a lower current is applied to the first light emitting diode module 100 while a higher current is applied to the second light emitting diode module 200.

As described above, the artificial solar light system of the present invention can correspond to changes in properties of the sun with time and to the travel property of the sun by employing light emitting diodes having a color temperature similar to that of the solar light and providing the control unit 600 for controlling the light emitting diodes. Further, since it is not required to provide an optical lens, which should have been installed outdoors to receive solar light, and an optical fiber for transmitting the solar light received by the optical lens, the artificial solar light system can be operated as desired regardless of changes in climate and can also be easily moved and managed. Accordingly, an expensive component such as the optical fiber is not required, and thus, there is no optical transmission loss due to the optical fiber.

According to the artificial solar light system using the light emitting diodes described above, it is possible to construct a light source for representing a spectrum similar to that of real solar light, and the light source can be applied to bio-industry, plant growth in indoor and underground environments, agriculture, and a region with insufficient sunshine such as an aquarium in which high-grade fish is brought up and displayed. Further, the artificial solar light system can be applied to illumination equipment for a dermatological patient, facilities such as an intensive care unit for a patient who cannot easily move, and the like. Moreover, the artificial solar light system can be applied to indoor decoration for image design, a psychical cure, beauty treatment, medical treatment, and the like.

Next, an artificial solar light system using light emitting diodes according to a second embodiment of the present invention, which includes a global positioning system (GPS), will be described with reference to the drawings. Portions of this embodiment overlapping with those of the previous embodiment will not be described or will be briefly described.

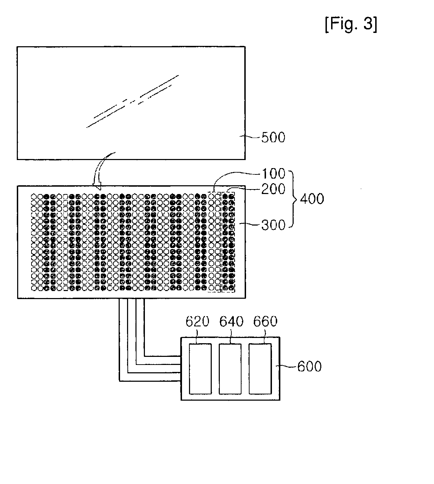

Fig. 3 is a conceptual view showing the artificial solar light system using light emitting diodes according to the second embodiment of the present invention.

As shown in Fig. 3, the artificial solar light system using light emitting diodes according to the second embodiment of the present invention includes a light source module 400, a diffusion globe 500 provided on the light source module 400, and a control unit 600 having a global positioning system 660 for controlling the light source module 400. At this time, the diffusion globe 500 may be eliminated, if necessary.

The control unit 600 is to operate the light source module 400, and includes a memory unit 640 for storing data on changes in the color temperature and optical properties of the sun with time, a microprocessor 620 for controlling the respective light emitting diode modules with different color temperatures by using the data, and the global positioning system 660 for identifying the position of the artificial solar light system.

That is, the artificial solar light system employs the global positioning system 660 to detect a position where the artificial solar light system is installed. With the detection of the position, the microprocessor 620 performs control of currents for the respective light emitting diode modules so that the artificial solar light system can correspond to changes in the color temperature and optical properties of the sun according to time and installation positions. The current control allows the light source module 400 to emit light having properties similar to changes in a spectrum of real solar light in a region where the artificial solar light system is installed.

When the global positioning system 660 is employed in the artificial solar light system as described above and the memory unit 640 stores solar coordinate system informations according to periods of earth's rotation and revolution, the artificial solar light system of the present invention has solar light emission properties which meet regional features all over the world. Since the solar light emission properties are changed depending on the latitude and longitude of a required region, for example, the winter in the northern hemisphere of the earth corresponds to the summer in the southern hemisphere thereof, the operational properties of the artificial solar light system are required to meet the solar light emission properties, As complementary measures for meeting the requirements, the global positioning system 660 which is inexpensive and of which a radio frequency channel is open all over the world is provided, the memory unit 640 stores the travel path of the sun (solar light emission properties) according to position information over the world and position information on a corresponding region, and the microprocessor 620 operates the light source module 400, as described above. Accordingly, it is possible to make more effective artificial solar light optimized to environments. However, the artificial solar light system of this embodiment is not limited thereto but may be operated to irradiate natural light of an environment to which a user is accustomed. That is, for example, if solar light similar to that in the spring in Korea is intended to be irradiated in the Arctic zone, the function of the global positioning system 660 is suspended and the latitude and longitude of Korea in March are inputted as the position information, so that artificial solar light corresponding thereto can be irradiated. Further, since it is not easy for people to comfortably receive solar light energy outdoors in these days from serious environmental pollution all over the world, the installation and operation of the artificial solar light system of the present invention on the ceiling of a desired room allows a user to safely receive light irradiation, which is similar to outdoor solar light in an indoor environment.

## Claims

1. An artificial solar light system using light emitting diodes, comprising: a plurality of light emitting diode modules (100, 200), the light emitting diode modules having different color temperatures and a plurality of light emitting diodes; and a control unit (600) for controlling the plurality of light emitting diode modules(100, 200),
the control unit (600) comprising:
a memory unit (640) configured to store data on changes in a color temperature and optical properties of the sun with time; and
a microprocessor (620) configured to control currents applied to the respective light emitting diode modules (100, 200) having different color temperatures by using the data on changes in the color temperature and optical properties of the sun with time, which have been stored in the memory unit (640), wherein
the memory unit (640) further includes solar coordinate system information according to periods of earth's rotation and revolution, and
the microprocessor (620) is configured to control the light emitting diode modules (100, 200) by using the data on changes in the color temperature and optical properties of the sun with time and the solar coordinate system information, which have been stored in the memory unit (640),
**characterized in that**
the control unit (600) further comprises a global positioning system (660) for identifying a position of the artificial solar light system; and the microprocessor (620) is configured to control the light emitting diode modules (100, 200) by using the data on changes in the color temperature and optical properties of the sun with time and the solar coordinate system information, which have been stored in the memory unit (640), depending on the position identified by the global positioning system (660).

2. The artificial solar light system as claimed in claim 1, further comprising a diffusion globe (500) arranged in front of the plurality of light emitting diode modules (100, 200) so as to mix light emitted from the plurality of light emitting diode modules (100, 200) and to irradiate the mixed light.

3. The artificial solar light system as claimed in claim 2, wherein the plurality of light emitting diode modules (100, 200) are alternately arranged.

4. The artificial solar light system as claimed in claim 1, wherein the plurality of light emitting diode modules (100, 200) have a color temperature of 3000K to 7000K.

5. The artificial solar light system as claimed in claim 6, wherein the microprocessor (620) receives information on a position and controls the light emitting diode modules (100, 200) by using the data on changes in the color temperature and optical properties of the sun with time and the solar coordinate system information, which have been stored in the memory unit (640), depending on the position information.

## Patentansprüche

1. Künstliches Tageslichtsystem unter Verwendung lichtemittierender Dioden, umfassend: eine Vielzahl lichtemittierender Diodenmodule (100, 200), wobei die lichtemittierenden Diodenmodule unterschiedliche Farbtemperaturen und eine Vielzahl lichtemittierender Dioden aufweisen; und eine Steuereinheit (600) zur Steuerung der Vielzahl lichtemittierender Diodenmodule (100, 200),
wobei die Steuereinheit (600) Folgendes umfasst:
eine Speichereinheit (640), die so konfiguriert ist, dass sie Daten in Bezug auf zeitliche Änderungen in der Farbtemperatur und den optischen Eigenschaften der Sonne speichert; und
einen Mikroprozessor (620), der so konfiguriert ist, dass er die an die entsprechenden lichtemittierenden Diodenmodule (100, 200) mit unterschiedlichen Farbtemperaturen angelegten Ströme steuert, wobei die in der Speichereinheit (640) gespeicherten Daten in Bezug auf zeitliche Änderungen in der Farbtemperatur und den optischen Eigenschaften der Sonne verwendet werden, wobei die Speichereinheit (640) weiterhin Information über Sonnenkoordinaten gemäß den Umlauf- und Rotationszeiten der Erde umfasst, und
der Mikroprozessor (620) so konfiguriert ist, dass er die lichtemittierenden Diodenmodule (100, 200) steuert, wobei die in der Speichereinheit (640) gespeicherten Daten in Bezug auf zeitliche Änderungen in der Farbtemperatur und den optischen Eigenschaftender Sonne und die Information über die Sonnenkoordinaten verwendet werden,
dadurch charakterisiert, dass
die Steuereinheit (600) weiterhin ein globales Positionsbestimmungssystem (660) zur Bestimmung einer Position des künstlichen Tageslichtsystems umfasst; und der Mikroprozessor (620) so konfiguriert ist, dass er die lichtemittierenden Diodenmodule (100, 200) steuert, wobei die in der Speichereinheit (640) gespeicherten Daten in Bezug auf zeitliche Änderungen in der Farbtemperatur und den optischen Eigenschaften der Sonne und die Information über die Sonnenkoordinaten verwendet werden.

2. Künstliches Tageslichtsystem, wie es in Anspruch 1 beansprucht wird, weiterhin umfassend ein gewölbtes Diffusorelement (500), das auf der Vielzahl lichtemittierender Diodenmodule (100, 200) so angeordnet ist, dass es das von der Vielzahl lichtemittierender Diodenmodule (100, 200) emittierte Licht mischt und das gemischte Licht abstrahlt.

3. Künstliches Tageslichtsystem, wie es in Anspruch 1 beansprucht wird, wobei die Vielzahl lichtemittierender Diodenmodule (100, 200) abwechselnd angeordnet sind.

4. Künstliches Tageslichtsystem, wie es in Anspruch 1 beansprucht ist, wobei die Vielzahl lichtemittierender Diodenmodule (100, 200) eine Farbtemperatur von 3000 K bis 7000 K aufweisen.

5. Künstliches Tageslichtsystem, wie es in Anspruch 6 beansprucht wird, wobei der Mikroprozessor (620) Information über eine Position erhält und die lichtemittierenden Diodenmodule (100, 200) steuert, indem er die in der Speichereinheit (640) gespeicherten Daten in Bezug auf zeitliche Änderungen in der Farbtemperatur und den optischen Eigenschaften der Sonne und die Information über die Sonnenkoordinaten verwendet.

## Revendications

1. Système de lumière solaire artificielle utilisant des diodes électroluminescentes, comprenant : une pluralité de modules de diode électroluminescente (100, 200), les modules de diode électroluminescente ayant différentes températures de couleur et une pluralité de diodes électroluminescentes ; et une unité de commande (600) pour commander la pluralité de modules de diode électroluminescente (100, 200),
l'unité de commande (600) comprenant :
une unité de mémoire (640) configurée pour stocker des données sur des changements d'une température de couleur et de propriétés optiques du soleil dans le temps ; et
un microprocesseur (620) configuré pour commander des courants appliqués aux modules de diode électroluminescente respectifs (100, 200) ayant différentes températures de couleur en utilisant les données sur des changements de la température de couleur et des propriétés optiques du soleil dans le temps qui ont été stockées dans l'unité de mémoire (640), dans lequel l'unité de mémoire (640) inclut en outre des informations de système de coordonnées solaires en fonction de périodes de rotation et de révolution de la terre, et
le microprocesseur (620) est configuré pour commander les modules de diode électroluminescente (100, 200) en utilisant les données sur des changements de la température de couleur et des propriétés optiques du soleil dans le temps et les informations de système de coordonnées solaires qui ont été stockées dans l'unité de mémoire (640),
**caractérisé en ce que**
l'unité de commande (600) comprend en outre un système de positionnement global (660) pour identifier une position du système de lumière solaire artificielle ; et le microprocesseur (620) est configuré pour commander les modules de diode électroluminescente (100, 200) en utilisant les données sur des changements de la température de couleur et des propriétés optiques du soleil dans le temps et les informations de système de coordonnées solaires qui ont été stockées dans l'unité de mémoire (640) en fonction de la position identifiée par le système de positionnement global (660).

2. Système de lumière solaire artificielle selon la revendication 1, comprenant en outre un globe de diffusion (500) agencé devant la pluralité de modules de diode électroluminescente (100, 200) de manière à mélanger de la lumière émise depuis la pluralité de modules de diode électroluminescente (100, 200) et à irradier la lumière mélangée.

3. Système de lumière solaire artificielle selon la revendication 2, dans lequel la pluralité de modules de diode électroluminescente (100, 200) est agencée en alternance.

4. Système de lumière solaire artificielle selon la revendication 1, dans lequel la pluralité de modules de diode électroluminescente (100, 200) a une température de couleur de 3000K à 7000K.

5. Système de lumière solaire artificielle selon la revendication 6, dans lequel le microprocesseur (620) reçoit des informations sur une position et commande les modules de diode électroluminescente (100, 200) en utilisant les données sur des changements de la température de couleur et des propriétés optiques du soleil dans le temps et les informations de système de coordonnées solaires qui ont été stockées dans l'unité de mémoire (640) en fonction des informations de position.
